# EUROPEAN PATENT APPLICATION

(11) **EP 4 843 548 A1**
(43) Date of publication of application: **25.11.2026**
(21) Application number: 26167580.5
(22) Date of filing: 25.03.2026
(51) Int. Cl.: A61F 13/15, A61F 13/56

(54) **CLOSURE ELEMENT, METHOD AND MACHINE FOR THE PRODUCTION OF ABSORBENT SANITARY ARTICLES AND RELATED ABSORBENT SANITARY ARTICLE**

(30) Priority: 23.05.2025 IT 202500011830
(71) Applicant: Fameccanica.Data S.p.A., 66020 San Giovanni Teatino (CH) (IT)
(72) Inventor: SABLONE, Gabriele, I-66020 San Giovanni Teatino (Chieti) (IT)
(74) Representative: Buzzi, Notaro & Antonielli d'Oulx S.p.A.

(57) **Abstract**

A closure element for the production of reclosable absorbent sanitary articles, comprising a nonwoven web (12) folded in a general omega configuration.

## Description

### Field of the invention

This invention relates to a closure element intended to be used for the production of absorbent sanitary articles.

The invention has been specifically developed for the production of reclosable absorbent sanitary articles, such as diapers, pant diapers, absorbent articles for incontinent adults, etc.

The invention is usable in particular in production processes called "*Cross Direction*"*.*

The invention also relates to an absorbent sanitary article produced using such a closure element.

According to another aspect the invention relates to a method and a machine for the production of absorbent sanitary articles.

### Prior art

A well-established technique for the production of reclosable absorbent sanitary articles involves forming a continuous composite tape comprising at least one continuous waistband, parallel to the machine direction, and a plurality of absorbent cores affixed to the continuous waistband and extending orthogonally to the machine direction. In certain cases, two continuous waistbands parallel to the machine direction are used, between which the absorbent cores are affixed. Sometimes the production process involves the use of a single continuous waistband parallel to the machine direction, to which the ends of absorbent cores extending orthogonally to the machine direction are affixed.

This production technique, called *Cross Direction,* is distinguished from the production technique known as *Machine Direction,* which involves producing absorbent sanitary articles as they advance with their longitudinal axes parallel to the machine direction.

Examples of methods for the production of absorbent sanitary articles according to the *Cross Direction* technique are described in documents EP-A-1013251, IT1379452, IT1410464, and IT1410465 by the same Applicant.

For the production of reclosable absorbent sanitary articles, reclosable closure elements spaced apart in the machine direction are applied to one of the continuous waistbands.

EP1523968-A by the same applicant describes a closure element for the production of absorbent sanitary articles wearable as panties, comprising a nonwoven fabric web folded in a general omega-shaped configuration and including a base divided into two sections located on opposite sides with respect to a central plane, two intermediate flaps located on opposite sides of the central plane, connected to respective outer ends of the base sections and permanently affixed to the base, two distal flaps located on opposite sides of the central plane, connected to respective inner ends of the respective intermediate flaps by respective folds and detachably affixed to respective intermediate flaps. The base of the closure element is affixed to a waistband of a sanitary article. Respective surface fastening elements are affixed on surfaces of respective distal flaps opposite to the base.

One of the problems with this solution is that the surface fastening elements (formed, for example, by micro-hook elements like Velcro^{©}) during the production process are exposed on a surface of the waistband.

This solution is suitable in cases where the absorbent sanitary articles are closed in the form of panties before packaging. In this case, during the production process, the absorbent sanitary articles are folded to bring the surface fastening elements of the closure elements into contact with corresponding closure elements to close the absorbent sanitary articles in the form of panties.

However, when the absorbent sanitary articles are intended to be packaged in an open configuration and to be closed when the article is worn, the surface fastening elements exposed on the waistbands tend to stick to any fabric surface or similar both during the production and packaging process and when the article is worn.

US2003078558A1 describes an absorbent sanitary article having a rear waistband provided with two closure elements, each comprising a nonwoven support having a distal portion provided with micro-hook fastening elements and a proximal portion that is permanently affixed to the outer surface of the rear waistband. Each of the closure elements can be folded a number of times, and the folds are held in place by glue or thermal or ultrasonic welding. To avoid contact between the micro-hook material and the wearer's clothing, the micro-hook fastening portion can be folded inward so that the micro-hook surface adheres to the surface of the nonwoven support.

The main disadvantage of the solution according to US2003078558A1 is that in the production process, two separate closure elements must be applied to each absorbent sanitary article, which complicates the equipment and the production process.

### Object and summary of the invention

The object of the present invention is to provide a closure element that solves the problems of the prior art.

According to the present invention, this object is achieved by a closure element having the features forming the subject of claim 1. According to another aspect, the invention relates to a method for the production of absorbent sanitary articles having the features forming the subject of claim 8.

Preferred embodiments form the subject of the dependent claims.

The claims form an integral part of the teaching provided in relation to the invention.

### Brief description of the drawings

The present invention will now be described in detail with reference to the attached drawings, provided purely by way of non-limiting example, in which:
- Figure 1 is a schematic perspective view of a closure element according to the present invention,
- Figure 2 is a schematic front view of the closure element of Figure 1,
- Figure 3 is a schematic plan view illustrating a step of a method for the production of absorbent sanitary articles,
- Figure 4 is a schematic front view of the part indicated by arrow IV in Figure 3,
- Figures 5 and 6 are schematic plan views of an absorbent sanitary article, with lateral closures respectively in the closed and open positions,
- Figure 7 is a schematic cross-section along line VII-VII of Figure 6, and
- Figures 8-12 are schematic plan views illustrating different embodiments of lateral closures indicated by arrow VIII of Figure 6.

### Detailed description

Referring to the figures, 10 denotes a closure element for the production of reclosable absorbent sanitary articles.

The closure elements 10 are intended to be affixed at spaced positions on a continuous elastic band in a process for the production of absorbent sanitary articles according to the technique called *Cross Direction,* as described in EP-A-1523968 by the same applicant.

The closure element 10 comprises a nonwoven web 12 folded in a general omega-shaped configuration to form a base 14, two intermediate flaps 18, and two distal flaps 24.

The base 14 is generally flat and is divided into two sections 16 located on opposite sides with respect to a central plane X orthogonal to the base 14.

The two intermediate flaps 18 are parallel to the base 14 and are located on opposite sides of the central plane X. The intermediate flaps 18 are connected to respective ends of the base 14 by respective first folds 20. The intermediate flaps 18 have respective surfaces 40 facing the base 14 and permanently affixed to corresponding surfaces 42 of the base 14, for example by respective layers of glue 22 or, alternatively, by thermal or ultrasonic welding.

The two distal flaps 24 are parallel to their respective intermediate flaps 18 and are located on opposite sides of the central plane X. The distal flaps 24 are connected to respective ends of the intermediate flaps 18 by respective second folds 26.

The closure element 10 includes surface fastening elements 30 arranged on surfaces 46 of the intermediate flaps 18 facing respective distal flaps 24.

The surface fastening elements 30 can be micro-hook fasteners capable of forming a Velcro^{®}-type surface connection with corresponding micro-loop fasteners.

The surface fastening elements 30 can be affixed to surfaces 46 of the intermediate flaps 18 by glue or by thermal or ultrasonic welding.

The surface fastening elements 30 can be integrally formed on surfaces 46 of the distal flaps 24, for example by an ultrasonic device of the type described in patent application EP4108220 by the same Applicant.

For each intermediate flap 18, the surface fastening elements 30 can be continuous along a direction parallel to the central plane X.

Alternatively, for each intermediate flap 18, the surface fastening elements 30 can be discontinuous along a direction parallel to the central plane X.

The surface fastening elements 30 can engage in a detachable manner surfaces 44 of respective distal flaps 24 facing the base 14. This is particularly useful in cases where the surface fastening elements 30 are micro-hook fasteners that engage the surfaces 44 of the distal flaps 24 made of nonwoven fabric.

Each of the surface fastening elements 30 is contained between a respective first fold 20 and a respective second fold 26. Thus, the surface fastening elements 30 do not protrude laterally beyond the respective first folds 20, thereby avoiding any contact between the surface fastening elements 30 and elements foreign to the closure element 10.

The distal flaps 24 may have respective ends 48 that protrude laterally beyond corresponding outer ends 50 of respective surface fastening elements 30. This facilitates the gripping with fingers of the ends 48 to open the closure element 10.

In possible embodiments, the ends 48 of the distal flaps 24 may protrude laterally outward beyond respective second folds 26 to further facilitate the gripping with fingers of the ends 48.

In possible embodiments, the surface fastening elements 30 can be adhesive fasteners capable of forming a reclosable adhesive-type surface connection to close the absorbent sanitary article 72 around the wearer's waist. In particular, the adhesive surface fastening elements 30 can be of the "stick and re-stick" type and/or repositionable so that the absorbent article can be repeatedly reclosed around the wearer's waist.

In general, any known method in the field of absorbent sanitary article production for forming a reclosable surface connection is suitable for the implementation of the invention described herein, such as the use of buttons and/or clips.

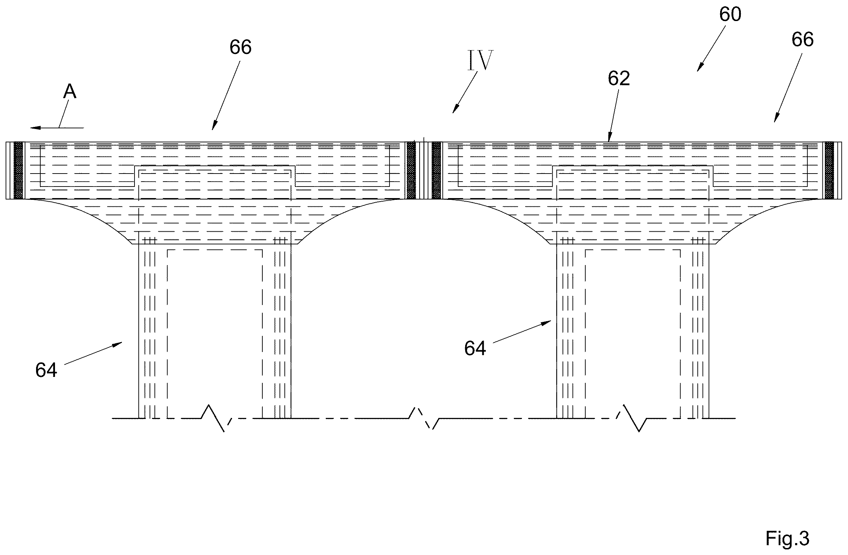

Figure 3 schematically illustrates a step of a method for the production of absorbent sanitary articles.

The method involves forming a continuous composite tape 60 advancing in a machine direction A. The continuous composite tape 60 is formed by a continuous array of absorbent sanitary article blanks 66.

The continuous composite tape 60 includes a continuous waistband 62 parallel to the machine direction A. The continuous waistband 62 is typically elastic and may comprise a plurality of elastic threads affixed between two nonwoven webs.

The continuous composite tape 60 includes a plurality of absorbent cores 64 having respective ends affixed to the continuous waistband 62. The absorbent cores 64 extend orthogonally to the machine direction A and are spaced apart from each other in the machine direction A.

The absorbent sanitary article blanks 66 each comprise an absorbent core 64 and a respective section of the continuous waistband 62.

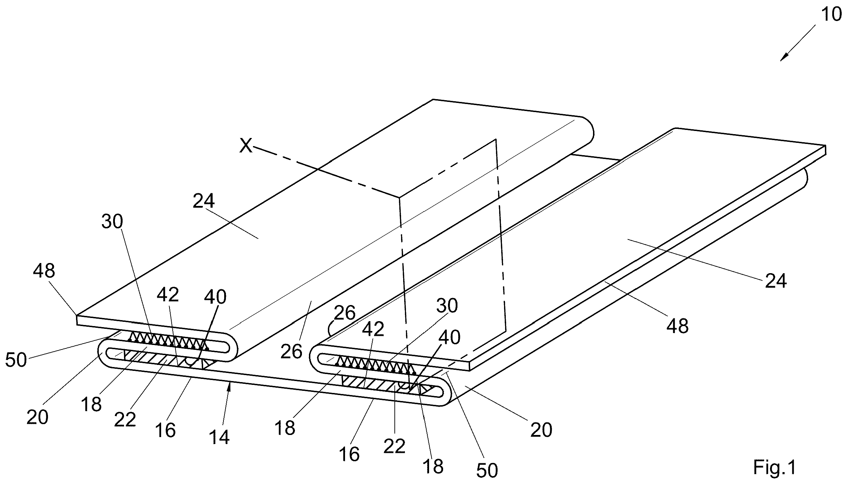

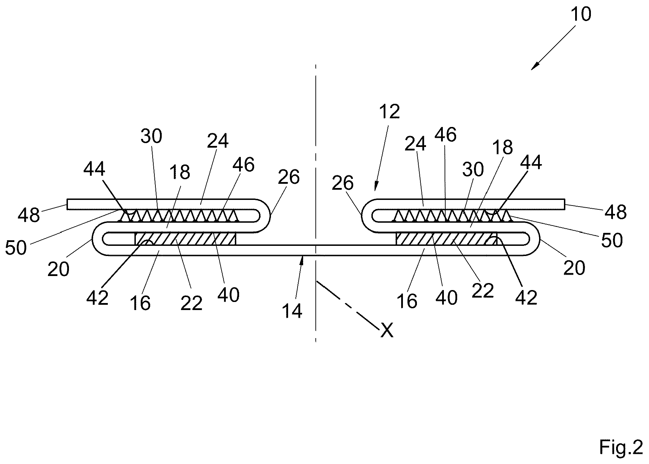

The method involves forming a plurality of fastening elements 10, each having the structure illustrated in Figures 1 and 2. The fastening elements 10 can be produced from a continuous nonwoven web, on two lateral regions of which respective continuous or intermittent tapes of micro-hook material forming the fastening elements 30 are affixed or integrally formed. Layers of glue 22 are also applied to the continuous nonwoven web. The continuous nonwoven web is folded into an omega shape to assume the form illustrated in Figures 1 and 2. Then, the continuous nonwoven web is cut transversely to form fastening elements 10.

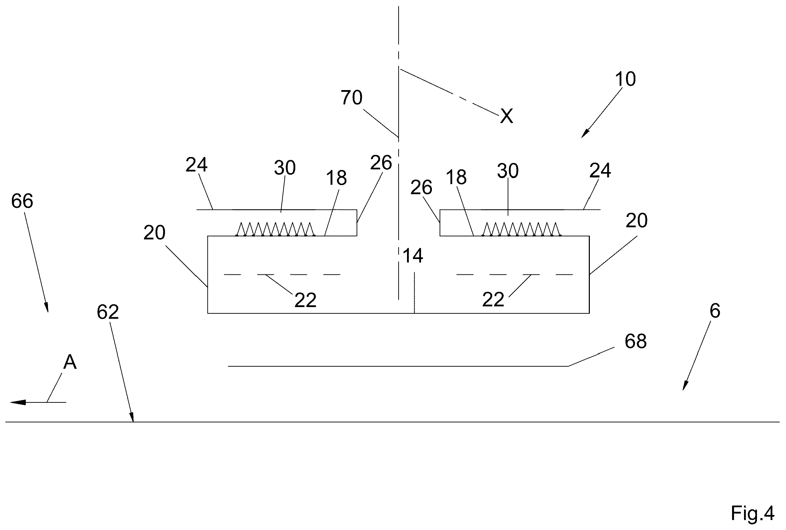

As illustrated in Figures 3 and 4, the bases 14 of the fastening elements 10 are affixed to a surface of the continuous waistband 62 at spaced positions along the machine direction A. As schematically shown in Figure 4, the affixing of the bases 14 of the fastening elements 10 to the continuous waistband 62 can be carried out, for example, by intermittent layers of glue 68 applied to the continuous waistband 62.

The fastening elements 10 are oriented with their central planes X orthogonal to the machine direction A. The fastening elements 10 are affixed to the continuous waistband 62 so that the central plane X of each closure element 10 is aligned with a separation line between each pair of adjacent absorbent sanitary article blanks 66.

As illustrated in Figure 3, the continuous waistband 62 may have an inner edge 80 with a shaped form to conform to the wearer's legs. The inner edge 80 may be cut flush with the inner edges of the surface fastening elements 30.

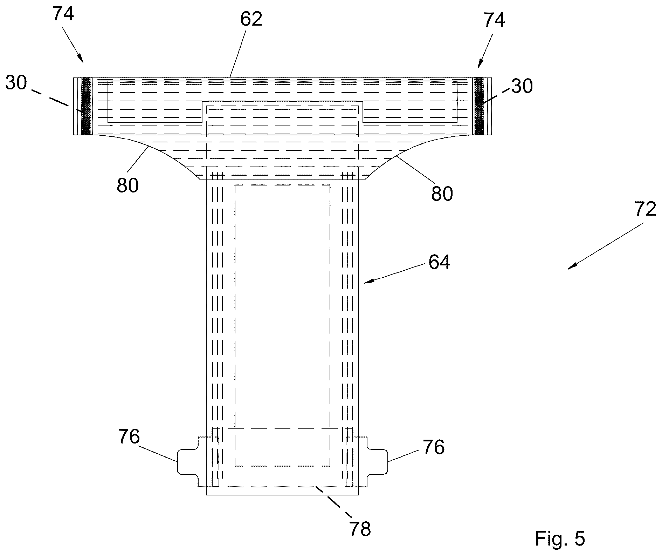

As illustrated in Figure 4, after the fastening elements 10 are affixed, the continuous waistband 62 is cut transversely along cutting lines 70 substantially aligned with the central planes X of the fastening elements 10. The transverse cutting of the continuous waistband 62 along the cutting lines 70 forms absorbent sanitary articles 72 having the shape schematically illustrated in Figure 5. Each cutting line 70 divides a respective closure element 10 into two symmetrical sections, each of which forms a lateral closure 74 of an absorbent sanitary article 72.

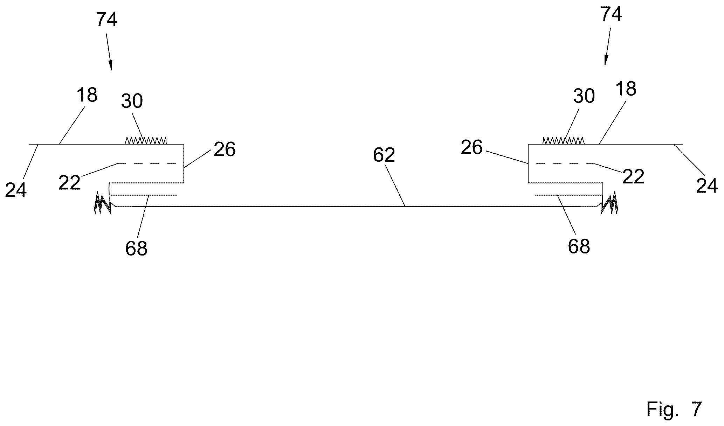

As illustrated in Figure 7, after the cutting of the continuous waistband 62, the portions of the continuous waistband 62 adjacent to the cutting line 70 retract elastically against their respective lateral closures 74.

During the forming, affixing, and cutting steps of the fastening elements 10, the surface fastening elements 30 are enclosed between respective distal flaps 24 and intermediate flaps 18 and engage in a detachable manner surfaces 44 of respective distal flaps 24 facing the base 14.

In this way, problems that could arise from the adherence of the surface fastening elements 30 to materials or components used in the production or packaging process of the absorbent sanitary articles 72 are avoided.

At the end of the production process, absorbent sanitary articles 72 are obtained, each comprising a waistband 62, an absorbent core 64 having one end affixed to the waistband 62, and two lateral closures 74 affixed to opposite ends of the waistband 62, where the lateral closures 74 are formed by respective sections of a closure element 10.

At the end of the production process, the absorbent sanitary articles 72 are folded and bagged to create product packs.

The absorbent sanitary articles 72 are packaged with the lateral closures 74 of the absorbent sanitary articles 72 in the closed configuration, in which the surface fastening elements 30 are enclosed between respective distal flaps 24 and intermediate flaps 18.

At the time of use, the surface fastening elements 30 are hidden and do not adhere to clothing or other fabric items or similar when the article is worn.

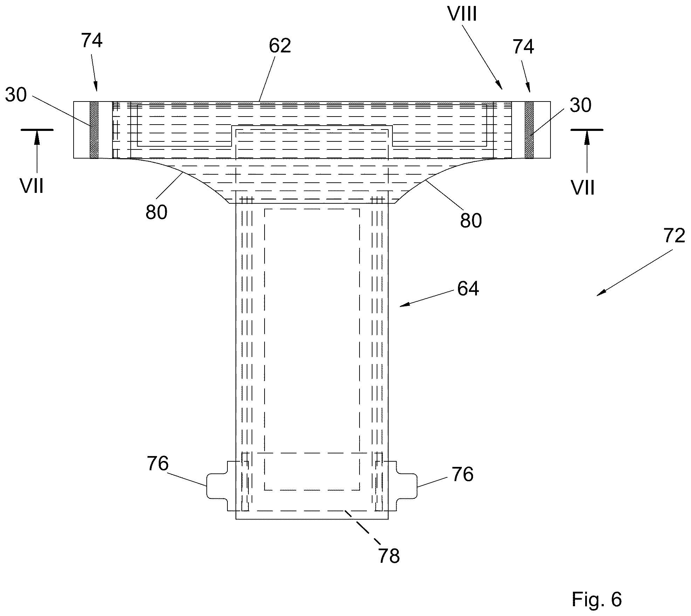

At the time of use, the lateral closures 74 are opened as illustrated in Figures 6 and 7. In this configuration, the surface fastening elements 30 are coupled with side tabs 76 or with a front panel 78 to close the absorbent sanitary article 72 around the wearer's waist.

The fact of affixing the surface fastening elements 30 of the intermediate flaps 18 allows for obtaining absorbent sanitary articles 72 in which the distance between the lateral closures 74 is particularly reduced, which is especially important in small-sized absorbent sanitary articles, for example, absorbent sanitary articles for infants. Indeed, infants have a very small waist circumference, and the distance between the lateral closures 74 must be slightly greater than the width of the absorbent core. The closure element according to the present invention allows for positioning the lateral closures 74 in the immediate vicinity of the lateral edges of the waistband 62 and thus allows for producing absorbent sanitary articles in which the distance between the lateral closures 74 is minimized compatible with the width of the absorbent core.

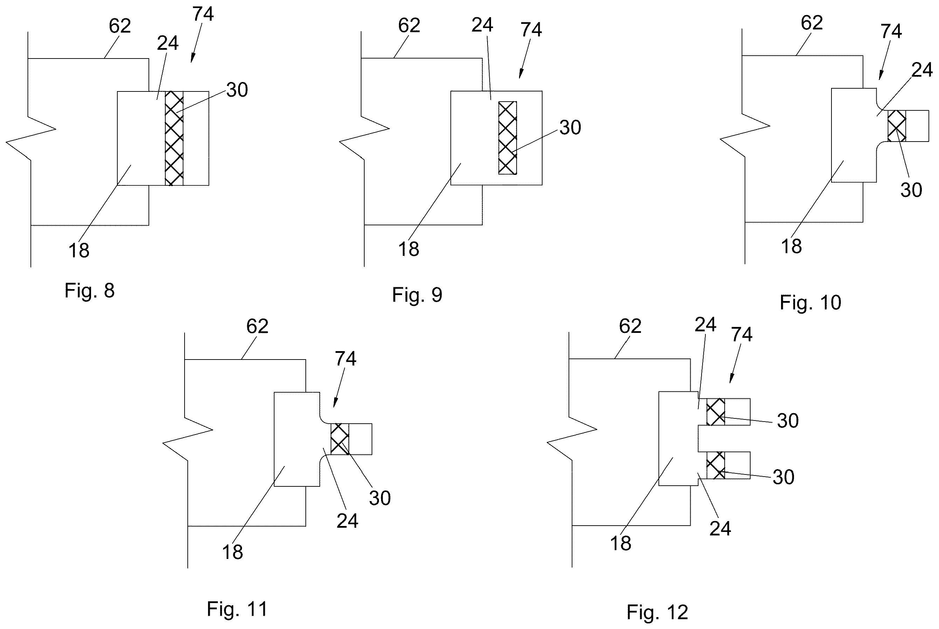

Referring to Figure 8, the surface fastening element 30 may have a width equal to that of the respective intermediate flap 18.

Alternatively, as illustrated in Figure 9, the surface fastening element 30 may have a width less than that of the respective intermediate flap 18 and may be surrounded on four sides by the material of the intermediate flap 18.

Referring to Figure 10, the distal flap 24 of each lateral closure 74 may be shaped to have a width less than that of the respective intermediate flap 18. The shaping may be asymmetrical as illustrated in Figure 11 (which involves a cut with waste).

Referring to Figures 11 and 12, the intermediate flap 18 of each lateral closure 74 may be cut symmetrically to form two lateral closures 74 with complementary shapes, thus avoiding the formation of waste.

To realize the embodiments of Figures 10, 11, and 12, it may be advantageous for the base 14 to be formed by two or more webs extending from opposite sides of the central plane X and joined together along a junction line parallel to the central plane X.

Of course, while the principle of the invention remains the same, the construction details and embodiment can be widely varied from what has been described and illustrated without departing from the scope of the invention as defined by the following claims.

## Claims

1. A closure element for the production of reclosable absorbent sanitary articles, comprising a nonwoven web (12) folded in a general omega configuration to form:
- a base (14) divided into two sections (16) located on opposite sides with respect to a central plane (X) orthogonal to the base (14),
- two intermediate flaps (18) parallel to the base (14) and located on opposite sides of said central plane (X), where the intermediate flaps (18) are connected to respective ends of the base (14) by respective first folds (20) and have respective surfaces permanently affixed to the base (14),
- two distal flaps (24) parallel to their respective intermediate flaps (18) and located on opposite sides of said central plane (X), where the distal flaps (24) are connected to respective ends of the respective intermediate flaps (18) by respective second folds (26),
wherein the closure element (10) includes surface fastening elements (30) arranged on respective surfaces (46) of the intermediate flaps (18) facing respective distal flaps (24).

2. The closure element of claim 1, wherein the surface fastening elements (30) are micro-hook surface fasteners.

3. The closure element of claim 1, wherein the surface fastening elements (30) are adhesive surface fasteners.

4. The closure element of any of the preceding claims, wherein the surface fastening elements (30) engage in a detachable manner surfaces (44) of respective distal flaps (24) facing the base (14).

5. The closure element of any of the preceding claims, wherein each of said surface fastening elements (30) is contained between a respective first fold (20) and a respective second fold (26).

6. The closure element of any of the preceding claims, wherein said distal flaps (24) have respective ends (48) that protrude laterally beyond corresponding outer ends (50) of respective surface fastening elements (30).

7. The closure element of claim 6, wherein said ends (48) of said distal flaps (24) protrude laterally outward beyond respective first folds (20).

8. An absorbent sanitary article comprising a waistband (62), an absorbent core (64) having one end affixed to said waistband (62), and two lateral closures (74) affixed to opposite ends of said waistband (62), wherein said lateral closures (74) are formed by respective sections of a closure element (10) according to any of the claims 1-7.

9. A method for the production of absorbent sanitary articles, comprising:
- forming a continuous composite tape (60) advancing in a machine direction (A) formed by a continuous array of absorbent sanitary article blanks (66), wherein the continuous composite tape (60) includes a continuous waistband (62) parallel to the machine direction (A) and a plurality of absorbent cores (64) having respective ends affixed to the continuous waistband (62),
- forming a plurality of fastening elements (10), according to any of the claims 1-7,
- affixing the bases (14) of the fastening elements (10) to a surface of the continuous waistband (62) at spaced positions along said machine direction (A), and
- cutting transversely said continuous waistband (62) along cutting lines (70) substantially aligned with the central planes (X) of the fastening elements (10).

10. The method of claim 9, wherein during said forming, affixing, and cutting steps of said fastening elements (10) the surface fastening elements (30) are enclosed between respective distal flaps (24) and intermediate flaps (18).
